# EUROPEAN PATENT APPLICATION

(11) **EP 2 510 876 A1**
(43) Date of publication of application: **17.10.2012**
(21) Application number: 12163192.3
(22) Date of filing: 04.04.2012
(51) Int. Cl.: A61B 5/1459, A61B 5/1455, A61B 1/06, A61B 1/00, A61B 1/04, A61B 5/00

(54) **Endoscopic diagnosis system**

(30) Priority: 11.04.2011 JP 2011087094
(71) Applicant: Fujifilm Corporation, Minato-ku Tokyo (JP)
(72) Inventor: Yamamoto, Hiroaki, Kanagawa (JP)
(74) Representative: Klunker . Schmitt-Nilson . Hirsch

(57) **Abstract**

An endoscopic diagnosis system includes a light source for simultaneously emitting at least two excitation light having different central wavelengths for exciting at least two autofluorescent substances to generate autofluorescence containing first autofluorescence whose intensity is strong in a lesion and weak in a normal region and second autofluorescence whose intensity is strong in a normal region and weak in a lesion, color filters having spectral transmission characteristics that block the excitation light and transmit the autofluorescence, image sensor for imaging autofluorescence generated from first and second autofluorescent substances contained in a subject's region under observation and dispersed by color filters to acquire first and second autofluorescence images as the at least two excitation light illuminate the region under observation, and an image sensor for image-processing and displaying the first and second fluorescence images.

## Description

### BACKGROUND OF THE INVENTION

The present invention relates to an endoscopic diagnosis system that images the autofluorescence emitted from autofluorescent substances contained in a subject's region under observation (organism) and obtains autofluorescence images.

There are conventionally used endoscope devices wherein normal light (white light) emitted from a light source device is guided to the tip of an endoscope to illuminate a subject's region under observation, and the reflected light is imaged to acquire a normal light image (white light image) for normal light observation (white light observation). In recent years, there have been used endoscope devices whereby a subject's region under observation is illuminated by excitation light for autofluorescence observation (special light), and the autofluorescence emitted from an autofluorescent substance is imaged to acquire an autofluorescence image (special light image) in order to perform autofluorescence observation (special light observation) in addition to normal light observation.

Among endoscope devices that perform autofluorescence observation is one described in JP 2008-43383 A.

JP 2008-43383 A describes a method of acquiring an autofluorescence image whereby in a first autofluorescence observation mode, three kinds of excitation light are sequentially emitted for excitation and cut off by an excitation light cut filter provided at the light receiving surface of an image sensor as the autofluorescence emitted from the autofluorescent substance is imaged by the image sensor to acquire an autofluorescence image. The three kinds of excitation light differ in wavelength and their wavelengths are optimized to capture autofluorescence images of NADH, elastin, collagen and other substances whose autofluorescence intensities vary between normal region and lesion.

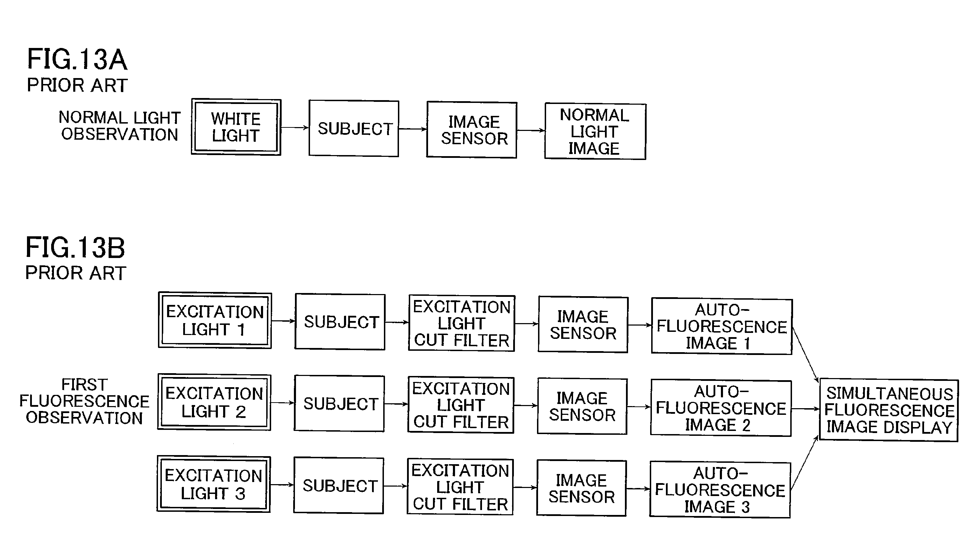

In normal light observation, the endoscope device described in JP 2008-43383 A illuminates a subject with white light and the reflected light thereof is imaged by the image sensor as illustrated in Fig. 13A. The captured image then undergoes processing that is appropriate for a normal light image before being displayed as a normal light image.

In autofluorescence observation, on the other hand, excitation light 1 first illuminates a subject, and autofluorescence corresponding to the excitation light 1 is imaged by the image sensor as the excitation light 1 is cut off by an excitation light cut filter as as illustrated in Fig. 13B. Then, the captured image undergoes processing that is appropriate for an autofluorescence image before an autofluorescence image 1 is produced. Excitation light 2 and 3 are then emitted for excitation to produce autofluorescence images 2 and 3 in a similar manner, and thereafter the autofluorescence images 1 to 3 are displayed simultaneously.

As illustrated in Fig. 14, JP 2008-43383 A describes a method whereby further in a second fluorescence observation mode, two excitation light 1 and 2 are sequentially emitted for excitation and cut off by an excitation light cut filter to image the autofluorescence and thereby obtain autofluorescence images 1 and 2, based on which the region where a bright area of the autofluorescence image 1 and a dark area of the autofluorescence image 2 coincide is turned into a specific color to display an autofluorescence image with the lesion enhanced.

### SUMMARY OF THE INVENTION

However, because the illumination timing differs among the three excitation light according to the method described in JP 2008-43383 A, the positions of the lesion fail to align among the three autofluorescence images even when the three fluorescence images are displayed simultaneously. In addition, three fluorescence images must be visually compared to determine the lesion, thus making the comparison difficult.

JP 2008-43383 A also describes measuring through simultaneous illumination with excitation light where, in the first fluorescence observation mode, the wavelengths of the excitation light are two wavelengths appropriate for capturing NADH and elastin. According to this method, however, distinction between normal region and lesion is difficult where, when NADH and elastin both emit fluorescence, the fluorescence intensity does not change in concordance between normal region and lesion.

Further still, JP 2008-43383 A describes a method of enhancing a lesion based on two autofluorescence images in the second fluorescence observation mode but the difference in illumination timing between the excitation light causes the position of the lesion to differ between the two autofluorescence images.

Thus, an object of the present invention is to provide an endoscopic diagnosis system enabling acquisition of autofluorescence images with no misalignment between the autofluorescence images permitting an accurate recognition of a lesion.

To achieve the above object, the present invention provides an endoscopic diagnosis system, comprising:
a light source for simultaneously emitting at least two excitation light having different central wavelengths for exciting at least two autofluorescent substances to generate autofluorescence, the at least two autofluorescent substances containing a first autofluorescent substance whose autofluorescence intensity is strong in a lesion and weak in a normal region and a second autofluorescent substance whose autofluorescence intensity is strong in a normal region and weak in a lesion;
color filters having spectral transmission characteristics that block the at least two excitation light and transmit the autofluorescence generated from the at least two autofluorescent substances;
image sensor for imaging autofluorescence generated, as the at least two excitation light simultaneously emitted from the light source illuminate a subject's region under observation, from the first and second autofluorescent substances contained in the region under observation and dispersed by the color filters to acquire first and second autofluorescence images; and
an image processor for image-processing the first and second autofluorescence images acquired by the image.sensor and displaying the image-processed first and second autofluorescence images on a monitor.

Preferably, the image processor assigns the first autofluorescence image to B and R channels and the second autofluorescence image to a G channel to display images displayed on the monitor in pseudo color.

Preferably, the endoscope diagnosis system further comprises a second light source for emitting white light;
second color filters having spectral transmission characteristics that transmit reflected light from the region under observation; and
a second image sensor for imaging the reflected light reflected from the region under observation and dispersed by the second color filters as white light emitted from the second light source illuminates the subject's region under observation to acquire a white light image;
wherein the image processor combines the image displayed on the monitor and the white light image acquired by the second image sensor and displays the combined image on the monitor.

Preferably, the light source emits two excitation light having central wavelengths of 405 nm and 445 nm.

Preferably, the excitation light are laser beams.

Preferably, the color filters comprise a green color filter that transmit light having a wavelength range of 500 nm to 600 nm and a red color filter that transmit light having a wavelength range of 600 nm to 700 nm.

Preferably, the first autofluorescent substance is porphyrin and the second autofluorescent substance is FAD.

According to the present invention, a plurality of excitation light having different wavelengths are emitted for simultaneous illumination, and the autofluorescence emitted from an autofluorescent substance is dispersed by color filters to acquire a first and a second autofluorescence image. Therefore, no misalignment occurs between the first and the second autofluorescence image, and the difference between normal region and lesion can be displayed with a high contrast, enabling an accurate distinction of a lesion. Another advantage is that no excitation light cut filer is required.

### BRIEF DESCRIPTION OF THE DRAWINGS

Fig. 1 is an external view of an embodiment illustrating a configuration of the endoscopic diagnosis system according to the invention.
Fig. 2 is a block diagram representing an internal configuration of the endoscopic diagnosis system of Fig. 1.
Fig. 3 is a conceptual view illustrating a tip portion of an endoscope insertion section of the endoscopic diagnosis system of Fig. 1.
Figs. 4A and 4B illustrate the concept of processing performed by the endoscopic diagnosis system shown in Fig. 1 in normal light observation mode and autofluorescence observation mode, respectively.
Fig. 5 illustrates the concept of operation of the endoscopic diagnosis system shown in Fig. 1.
Fig. 6 is a graph illustrating an example of light absorption intensity characteristics of the light emitted from an autofluorescent substance.
Fig. 7 is a graph illustrating an example of fluorescence intensity characteristics of autofluorescent substances.
Fig. 8 is a graph illustrating an example of fluorescence intensity characteristics of autofluorescent substances.
Fig. 9 is a graph where spectral transmission characteristics of color filters are superimposed over the graph of Fig. 7.
Fig. 10 is a graph where spectral transmission characteristics of color filters are superimposed over the graph of Fig. 8.
Fig. 11 is a graph where spectral transmission characteristics of color filters are superimposed over a graph of fluorescence intensity distribution of autofluorescence where a plurality of excitation light having different wavelengths are emitted for simultaneous illumination.
Fig. 12 is a table showing fluorescent intensities of FAD and porphyrin in a normal region and a lesion.
Figs. 13A and 13B are conceptual views illustrating processing performed in normal light observation mode and a first autofluorescence observation mode respectively according to the method described in JP 2008-43383 A.

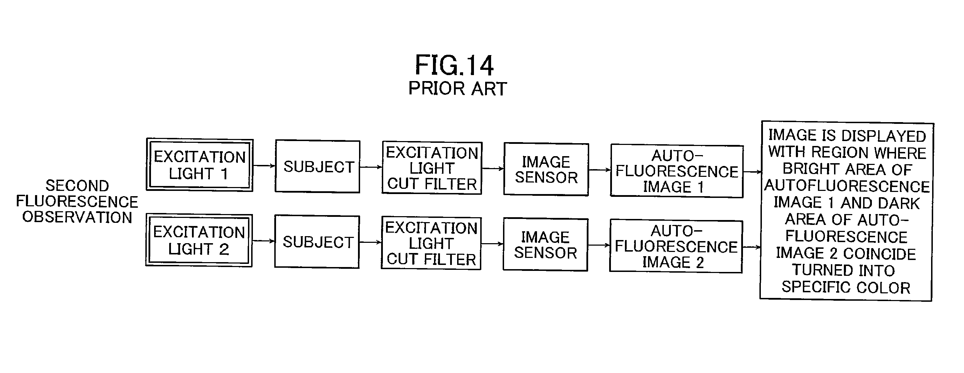

Fig. 14 is a conceptual view illustrating a second autofluorescence observation mode according to the method described in JP 2008-43383 A.

### DETAILED DESCRIPTION OF THE INVENTION

The endoscopic diagnosis system according to the present invention will be described below in detail based on the preferred embodiments illustrated in the attached drawings.

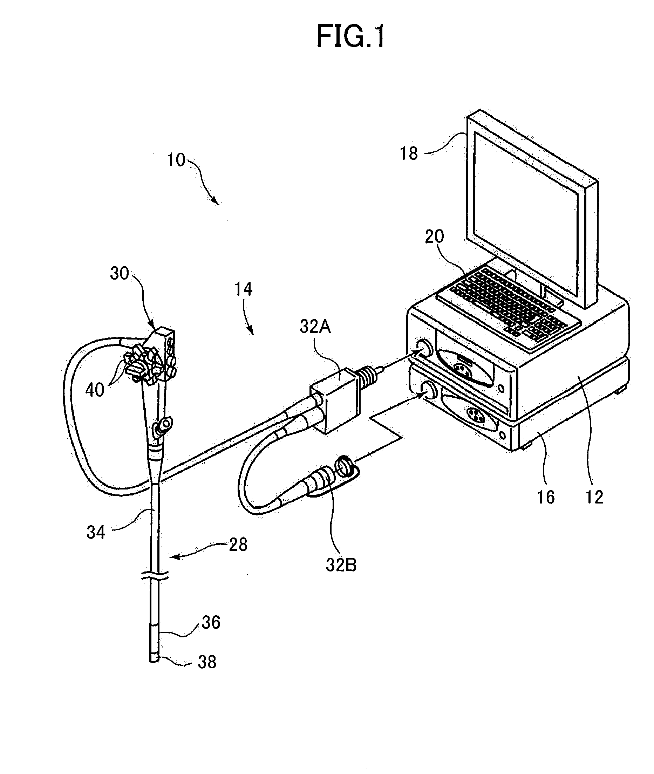

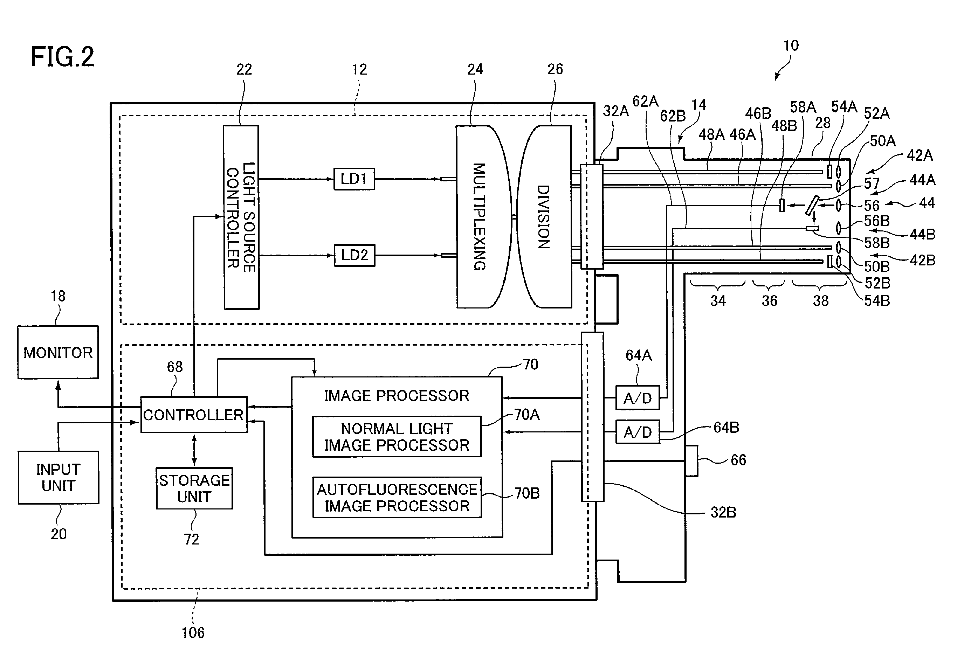

Fig. 1 is an external view of an embodiment illustrating a configuration of the endoscopic diagnosis system according to the invention; Fig. 2 is a block diagram illustrating an internal configuration thereof. An endoscopic diagnosis system 10 illustrated in these drawings comprises a light source device 12 for emitting a plurality of light having different ranges of wavelength; an endoscope device 14 for guiding light emitted from the light source device 12 to illuminate a subject's region under observation and image the reflected light or an autofluorescence from the subject; a processor 16 for image-processing the image acquired by the endoscope device 14 and outputting an endoscopic image; a monitor 18 for displaying the endoscopic image outputted from the processor 16; and an input unit 20 for receiving input operations.

The endoscopic diagnosis system 10 is capable of normal light observation mode (white light observation mode) for illuminating the subject with normal light (white light) and imaging the reflected light thereof to display (observe) a normal light image (white light image) and an autofluorescence observation mode (special light observation mode) for illuminating the subject with excitation light (special light) for autofluorescence observation and imaging the autofluorescence to display an autofluorescence image (special light image). The observation mode is switched as appropriate according to an instruction entered by a selector switch 66 of the endoscope device 14 or the input unit 20.

The light source device 12 comprises a light source controller 22, two kinds of laser light sources LD1, LD2 for emitting laser beams having different central wavelengths, a combiner (multiplexer) 24, and a coupler (demultiplexer) 26.

According to this embodiment, the laser light sources LD1, LD2 emit narrowband light beams having given wavelength ranges with central wavelengths of 405 nm and 445 nm (e.g., central wavelength +/- 10 nm), respectively. The laser light sources LD1, LD2 are light sources for emitting excitation light that causes autofluorescent substances such as, for example, porphyrin, NADH (reduced nicotinamide adenine dinucleotide), NADPH (reduced nicotinamide adenine dinucleotide phosphate) and FAD (flavin adenine dinucleotide) to emit autofluorescence. The laser light source LD2 also generates excitation light for causing a fluorescent body to generate white light (pseudo white light) as described later.

The on/off control and light amount control of the laser light sources LD1, LD2 are made independently from each other by the light source controller 22, which is in turn controlled by a controller of the processor 16 described later, and the emission timing and the light amount ratio can be freely varied. The laser light sources LD1, LD2 may be constituted using, among others, broad area type InGaN-based laser diodes as well as InGaNas-based laser diodes and GaNas-based laser diodes.

The normal light source for generating normal light is not limited to a light source using a combination of excitation light and fluorescent body and may be any light source to generate white light, including, for example, a xenon lamp, a halogen lamp, and a white LED (light emitting diode). The excitation light source for generating excitation light for autofluorescence observation is also not limited to a laser light source (semiconductor laser) and may be selected from various kinds of light sources capable of emitting excitation light having a sufficient intensity to excite an autofluorescent substance to emit autofluorescence, an example thereof being a combined use of a white light source and a band-limiting filter.

The excitation light for normal light observation is not specifically limited in wavelength (central wavelength or wavelength range of narrowband light) and may be any excitation light having a wavelength capable of causing a fluorescent body to generate pseudo white light. The excitation light for autofluorescence observation is also not specifically limited in wavelength and may be any excitation light having a wavelength capable of exciting an autofluorescent substance to emit autofluorescence; for example, light having a wavelength of 370 nm to 470 nm, in particular a wavelength of 400 nm to 450 nm, is preferably used.

Although the normal light source doubles as one of the excitation light sources in this embodiment, separate light sources may be provided instead.

The light source controller 22 turns the laser light source LD1 off and turns the laser light source LD2 on in the normal light observation mode. The light source controller 22 turns on both the laser light sources LD1 and LD2 in the autofluorescence observation mode.

The laser beams emitted from the laser light sources LD1, LD2 are passed through condenser lenses (not shown) to enter their respective optical fibers, combined by the combiner 24, and divided into four channels of light beams by the coupler 26 before being transmitted to a connector unit 32A. The combiner 24 and the coupler 26 are composed of, for example, a half mirror or a reflection mirror. The configuration is not limited this way; the laser beams from the laser light sources LD1, LD2 may be directly transmitted to the connector unit 32A in lieu of through the combiner 24 and the coupler 26.

The endoscope device 14 is an electronic endoscope device comprising an optical system for illumination for emitting four channels (four beams) of light (normal light or excitation light for autofluorescence observation) from the tip of the endoscope insertion section inserted into the subject's body and two channels (two sensors) of optical imaging system for acquiring an endoscopic image of a region under observation. The endoscope device 14 comprises the endoscope insertion section 28, an operating unit 30 for bending the tip of the endoscope insertion section 28 and performing operations for observation, and connectors 32A, 32B for detachably connecting the endoscope device 14 to the light source device 12 and the processor 16.

The endoscope insertion section 28 comprises a flexible portion 34 having a flexibility, a bending portion 36, and a tip (also referred to below as of the endoscope tip portion) 38.

The bending portion 36 is provided between the flexible portion 34 and the tip 38 and is so configured as to be bendable by rotating an angle knob 40 provided on the operating unit 30. The bending portion 36 can be bent in any direction and to any angle according to, for example, the subject's site under observation for which the endoscope device 14 is used, so that the endoscope tip portion 38 may be directed toward a desired site for observation.

The operating unit 30 and the endoscope insertion section 28 contain therein, a forceps channel for inserting, for example, a tissue collecting tool, air/water supply channels, and other channels, not shown.

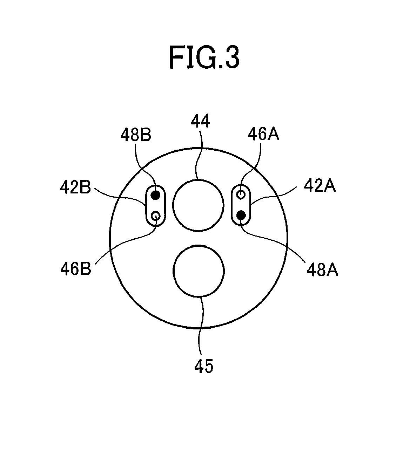

At the tip end surface of the endoscope tip portion 38 are provided, for example, two channels of illumination windows 42A, 42B for illuminating a region under observation and one channel of observation window 44 for imaging the reflected light or autofluorescence from the region under observation, as well as a forceps port 45 illustrated in Fig. 3.

On the inside of the illumination window 42A are provided two channels of optical fibers 46A, 48A. The optical fibers 46A, 48A extend from the light source device 12 through the connector unit 32A to the endoscope tip portion 38. The optical fiber 46A has at its tip portion (its end closer to the illumination window 42A) an optical system including, for example, a lens 50A. The optical fiber 48A has at its tip portion a fluorescent body 54A, and beyond the fluorescent body 54A is provided an optical system including, for example, a lens 52A.

On the inside of the illumination window 42B are likewise provided two channels of optical fibers: an optical fiber 46B having at its tip portion an optical system including, for example, a lens 50B and an optical fiber 48B having at its tip portion an optical system including, for example, a fluorescent body 54B and a lens 52B.

Fluorescent bodies 54A, 54B comprise a plurality of kinds of fluorescent substances that emit green to yellow light when excited upon absorbing part of the blue laser beam emitted from the laser light source LD2 (e.g., YAG-based fluorescent substances or fluorescent substances such as BAM (BaMgAl₁₀O₁₇)). When the excitation light for normal light observation illuminates the fluorescent bodies 54A, 54B, the green to yellow excited luminescence light (fluorescence) emitted from the fluorescent bodies 54A, 54B is allowed to blend with the blue laser beam that is transmitted without being absorbed by the fluorescent bodies 54A, 54B to generate white light (pseudo white light).

The sections of the optical system for illumination each comprising the illumination windows 42A and 42B have an equivalent configuration and operation. The illumination windows 42A, 42B simultaneously emit equivalent illumination light to preclude uneven illumination. Alternatively, the illumination windows 42A, 42B may emit different illumination light. Provision of an optical system for illumination that emits four channels of illumination light is not essential; an optical system for illumination to emit two or one channel of illumination light, for example, may be used to achieve an equivalent function.

On the inside of the observation window 44 is installed an optical system including, for example, a lens 56; behind the lens 56, a half mirror 57 is provided. Further down the optical path of the light transmitted through the half mirror 57 and the optical path of the light reflected by the half mirror 57 are provided image sensors 58A and 58B for acquiring image information on the region under observation, which may be, for example, a CCD (Charge Coupled Device) image sensor or a CMOS (Complementary Metal-Oxide Semiconductor) image sensor. The image sensor 58A is provided for normal light observation; the image sensor 58B is provided for autofluorescence observation. Because the fluorescent intensity of autofluorescence is feeble, the image sensor 58B for autofluorescence observation has a higher sensitivity than the image sensor 58A for normal light observation in this embodiment.

The received light may be directed otherwise than by means of the half mirror 57; a total reflection mirror, for example, may be placed on and retracted from the optical path of the received light to direct the received light to the image sensor 58A or 58B.

The image sensors 58A, 58B receive light from the lens 56 (transmitted light, reflected light) with their light receiving surface (imaging surface), perform photoelectric conversion of the received light, and output an image signal (analog signal). Three colors of pixels, a red pixel, a green pixel, and a blue pixel, form one set of pixels, and a plurality of sets of pixels are arranged in the form of matrix. The receiving surface of the image sensor 58A is provided with red, green, and blue color filters for the red pixels, the green pixels, and the blue pixels, the color filters having such spectral transmission characteristics as to transmit the reflected light from the region under observation having a wavelength ranging from about 370 nm to 720 nm of visible light by dividing the reflected light into three. The receiving surface of the image sensor 58B is provided with red and green color filters for the red pixels and the green pixels, the color filters having such spectral transmission characteristics as to transmit the autofluorescence emitted from the autofluorescent substances and having a wavelength ranging from about 500 nm to 700 nm while shielding the excitation light.

The light emitted from the light source device 12 and guided through the optical fibers 46A, 46B and 48A, 48B is emitted from the endoscope tip portion 38 toward the subject's region under observation. The reflected light from the illuminated region under observation or the autofluorescence emitted from the autofluorescent substances in the area under observation is focused by the lens 56 to form images on the light receiving surfaces of the image sensors 58A, 58B and undergoes photoelectric conversion through the image sensors 58A, 58B to yield images. The image sensors 58A, 58B output imaging signals (analog signals) of the subject's imaged region under observation.

In the normal light observation mode, the excitation light for normal light observation emitted from the light source LD2 is guided through the optical fibers 48A, 48B to illuminate the fluorescent bodies 54A, 54B, whereupon the white light generated from the fluorescent bodies 54A, 54B are emitted from the illumination windows 42A, 42B to illuminate the subject's region under observation. The reflected light from the subject's region under observation illuminated by the white light is condensed by the lens 56 and dispersed by the color filters, whereupon a normal light image is acquired by the image sensor 58A.

In the autofluorescence observation mode, the excitation light for autofluorescence observation emitted from both the laser light sources LD1 and LD2 are guided through the optical fibers 46A, 46B and emitted from the endoscope tip portion 38 toward the subject's region under observation. The autofluorescence emitted from the autofluorescent substances in the subject's region under observation illuminated by the excitation light is condensed by the lens 56 and dispersed by the color filters, whereupon a green and a red autofluorescence image are acquired by the image sensor 58B.

Imaging signals (analog signals) of images (normal light image and autofluorescence image) outputted from the image sensors 58A, 58B travel through scope cables 62A, 62B to enter A/D converters 64A, 64B. The A/D converters 64A, 64B convert the imaging signals (analog signals) supplied from the image sensors 58A, 58B to image signals (digital signals). The image signal obtained through the conversion passes through the connector unit 32B to enter an image processor 70 of the processor 16.

The processor 16 comprises the controller 68, the image processor 70, and a storage unit 72. The controller 68 is connected to the monitor 18 and the input unit 20. The processor 16 controls the light source controller 22 of the light source device 12 according to an instruction inputted from the selector switch 66 of the endoscope device 14 and the input unit 20 and image-processes image signals inputted from the endoscope device 14 to produce and output a display image to the monitor 18.

The controller 68 controls the operations of the image processor 70 and the light source controller 22 of the light source device 12 according to instructions, such as an observation mode instruction, given by the selector switch 66 of the endoscope device 14 and the input unit 20.

The image processor 70 performs a given image processing on the image signal entered from the endoscope device 14 under the control by the controller 68 according to the observation mode depending on the kinds of images including a normal light image and an autofluorescence image. The image processor 70 comprises a normal light image processor 70A and an autofluorescence image processor 70B.

In the normal light observation mode, the normal light image processor 70A performs a given image processing appropriate for a normal light image on the image signal (image data) of a normal light image supplied from the A/D converter 64A and outputs (produces) a normal light image signal (normal light image).

In the autofluorescence observation mode, the autofluorescence image processor 70B performs a given image processing appropriate for an autofluorescence image on the image signal (image data) of an autofluorescence image supplied from the A/D converter 64B and outputs (produces) an autofluorescence image signal (autofluorescence image). Because the intensity of the autofluorescence is feeble, the autofluorescence image processor 70B, for example, performs a given signal amplification processing.

The image signal processed by the image processor 70 is sent to the controller 68. The controller 68 causes the normal light image or a combined image of the normal light image and the autofluorescence image to be displayed on the monitor 18 according to the observation mode based on the normal light image signal and the autofluorescence image signal. Further, where necessary, the controller 68 causes the normal light image signal and the autofluorescence image signal to be stored in the storage unit 72, which may be configured by a memory or a storage device, in units of, for example, one sheet (one frame) of image.

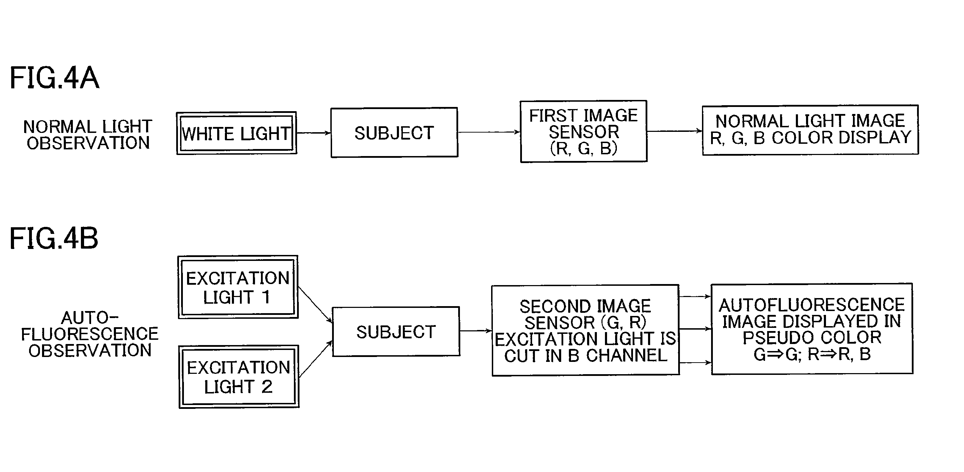

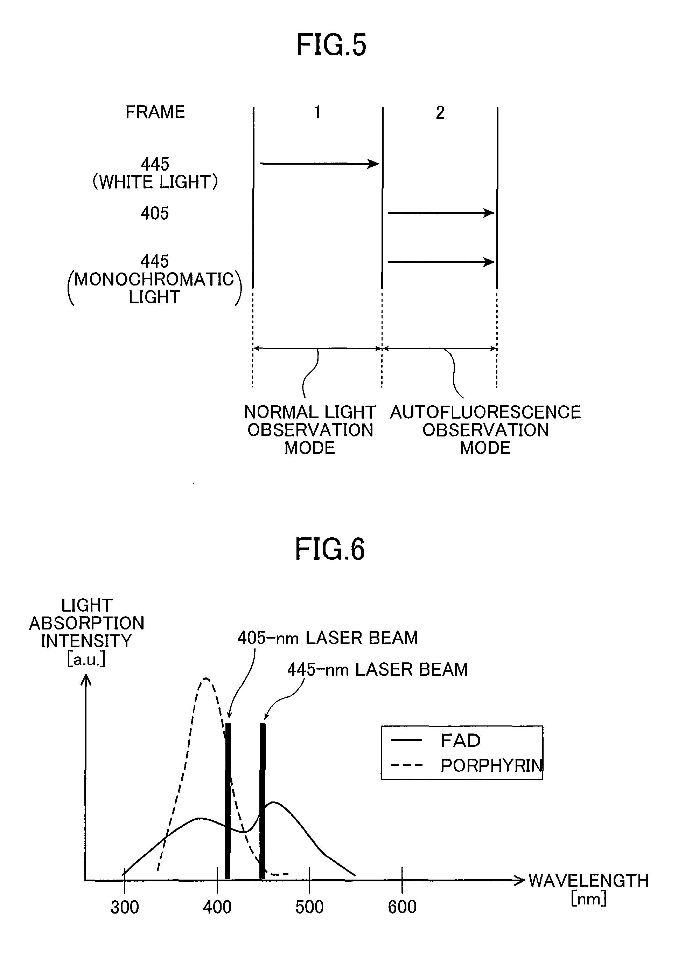

The operation of the endoscopic diagnosis system 10 will now be described referring to the conceptual views of Figs. 4A, 4B and Fig. 5.

In the normal light observation mode, the light source controller 22 controls the laser light source LD1 to be turned off and the laser light source LD2 to be turned on. The laser beam having a central wavelength of 445 nm emitted from the laser light source LD2 is allowed to illuminate the fluorescent bodies 54A, 54B, whereupon the fluorescent bodies 54A, 54B emit white light as illustrated in FIG. 5. The white light emitted from the fluorescent bodies 54A, 54B are allowed to illuminate the subject, and the reflected light thereof is received by the image sensor 58A (first image sensor) to acquire a normal light image containing R, G, and B channel image signals as illustrated in Fig. 4A. The normal light image is displayed in color based on the B, G, and R channels of image signals in normal light image processing.

In the autofluorescence observation mode, imaging process is repeated in units of, for example, two frames as illustrated in Fig. 5. The first of the two frames is in the same observation mode as the normal light observation mode; the second frame is in an observation mode unique to the autofluorescence observation mode.

First, in the normal light observation mode in the first frame, the normal light image containing the R, G, and B channels of image signals are acquired as described above. The normal light image signal is stored in the storage unit 72 under the control of the controller 68.

In the autofluorescence observation mode in the second frame, the light source controller 22 controls both the laser light sources LD1, LD2 to be turned on as illustrated in Fig. 5. As illustrated in Fig. 4B, the laser beam having a central wavelength of 405 nm emitted from the laser light source LD1 (excitation light) and the laser beam having a central wavelength of 445 nm simultaneously emitted from the laser light source LD2 (excitation light) illuminate the subject, whereupon the subject emits the autofluorescence, which is received by the image sensor 58B (second image sensor), which acquires an autofluorescence image containing image signals of the R and G channels. Then, the autofluorescence image is combined with the normal light image corresponding to the normal light image signal stored in the storage unit 72, and a synthesized image produced from these two images is displayed on the monitor 18. The autofluorescence image is displayed in pseudo color with the G channel image signal assigned to the G channel, and the R channel image signal assigned to the R channel and the B channel in autofluorescence image processor.

As described earlier, the image sensor 58B is not provided with blue color filters but provided only with green and red color filters. Therefore, excitation light having a wavelength range corresponding to the color of blue, i.e., central wavelengths of 405 nm and 445 nm, is cut off, so that the image sensor 58B receives only the autofluorescence in a wavelength corresponding to the colors of green and red, i.e., a wavelength range of 500 nm to 700 nm.

In the autofluorescence observation mode, repetition of imaging in units of two frames is not essential. In displaying the autofluorescence image in pseudo colors, an image signal of a channel of a specific color may be assigned to a channel of any of the specific colors as desired.

The relationship between the excitation light for autofluorescence observation and autofluorescence will now be described.

Fig. 6 is a graph illustrating an example of light absorption intensity characteristics of autofluorescent substances. In the graph, the vertical axis shows light absorption intensity of autofluorescent substances (a.u.: any unit), and the horizontal axis shows wavelength (nm). The graph shows absorption intensity characteristics of FAD and porphyrin, both autofluorescent substances. The graph also shows central wavelengths 405 nm and 445 nm of the laser beams used as excitation light for the autofluorescence observation in this embodiment.

FAD has characteristics to absorb light having a wavelength range of about 270 nm to 540 nm. The light absorption intensity of FAD gradually increases as the wavelength increases from about 270 nm, reaches a first peak at a wavelength of about 380 nm, and thereafter gradually decreases as the wavelength increases, reaching a minimum at a wavelength of about 420 nm. The absorption intensity then gradually increases again as the wavelength increases from about 420 nm and reaches a second peak at a wavelength of about 460 nm, thereafter gradually decreasing as the wavelength increases.

Porphyrin has characteristics to absorb light having a wavelength range of about 340 nm to 450 nm. Porphyrin has light absorption intensity characteristics peaking at a wavelength of about 390 nm and gradually decreasing as the wavelength decreases or increases.

As will be apparent from the graph, illumination of the subject with a laser beam having a central wavelength of 405 nm enables excitation of mostly porphyrin in the region under observation to generate autofluorescence. Illumination of the subject with a laser beam having a central wavelength of 445 nm enables excitation of mostly FAD in the region under observation to generate autofluorescence.

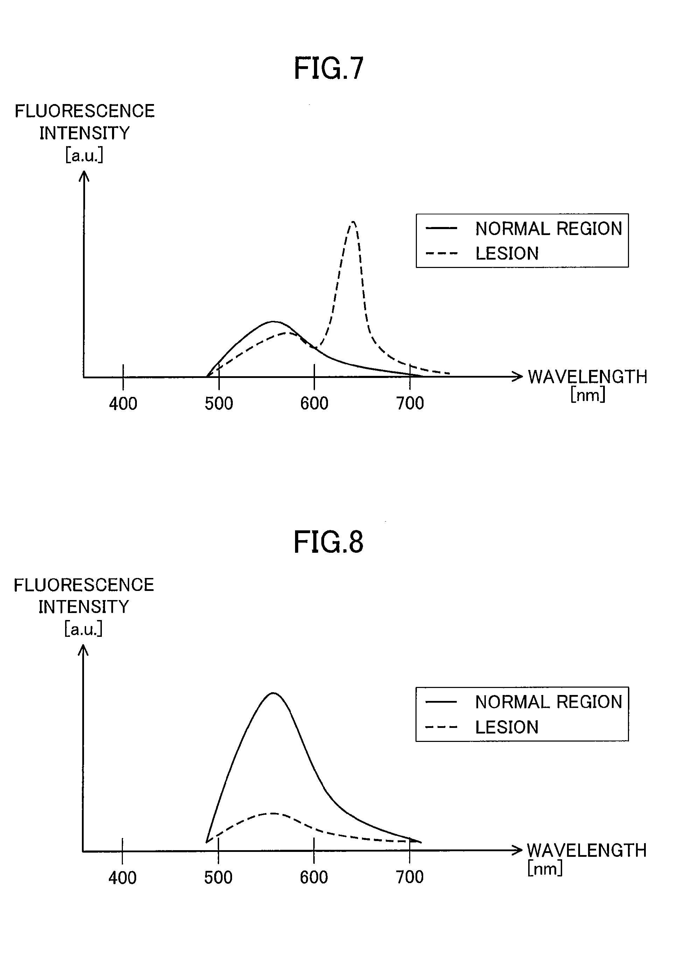

Fig. 7 is a graph illustrating an example of fluorescence intensity characteristics of autofluorescent substances. In the graph, the vertical axis shows fluorescence intensity of autofluorescent substances (a.u.), and the horizontal axis shows wavelength (nm). The graph, corresponding to the graph of Fig. 6, shows a fluorescence intensity distribution of autofluorescence emitted from the autofluorescent substance in the normal region and the lesion when the subject's region under observation is illuminated by laser beams having central wavelengths of 405 nm used as excitation light for the autofluorescence observation.

When the laser beam having a central wavelength of 405 nm is directed to illuminate the subject as excitation light for autofluorescence observation, mostly porphyrin is excited as described above and, as illustrated in Fig. 7, the region under observation illuminated by the excitation light generates autofluorescence having a wavelength range of about 480 nm to 740 nm.

The fluorescence intensity in the lesion gradually increases as the wavelength increases from about 480 nm, reaches a first peak at a wavelength of about 560 nm and thereafter gradually decreases as the wavelength increases, reaching a minimum at a wavelength of about 610 nm. The fluorescence intensity then gradually increases again as the wavelength increases from about 610 nm and reaches a second peak at a wavelength of about 630 nm, thereafter gradually decreasing as the wavelength increases. The second peak is exhibited by fluorescence emitted mostly from porphyrin.

On the other hand, the fluorescence intensity in the normal region gradually increases as the wavelength increases from about 480 nm and reaches a peak at a wavelength of about 550 nm, thereafter gradually decreasing as the wavelength increases.

It is generally known that in lesions, porphyrin accumulates. As illustrated in Fig. 7, the fluorescence intensity of porphyrin is higher in the lesion than in the normal region. Therefore, recognizing the difference in fluorescence intensity of porphyrin enables distinction between normal region and lesion (reference: "Hikari niyoru igakushindan, Vol. VI in the "Series / Hikari ga hiraku seimeikagaku" by Mamoru TAMURA, March 18, 2001, edited by Photobiology Association of Japan and published by KYORITSU SHUPPAN CO., LTD.).

The graph of Fig. 8 illustrates a fluorescence intensity distribution of autofluorescence emitted from autofluorescent substances in the normal region and the lesion as the subject's region under observation is illuminated by a laser beam having a central wavelength of 445 nm used as excitation light for autofluorescence observation.

When a laser beam having a central wavelength of 445 nm is allowed to illuminate the subject as excitation light for autofluorescence observation, FAD is mostly excited as described earlier, and, as illustrated in Fig. 8, the subject's region under observation illuminated by the excitation light generates autofluorescence having a wavelength range of about 480 nm to 720 nm.

The fluorescence intensities in both the lesion and the normal region gradually increase as the wavelength increases from about 480 nm and reach a peak at a wavelength of about 550 nm, thereafter gradually decreasing as the wavelength increases. The peak is reached by fluorescence emitted mostly from porphyrin.

As illustrated in the graph of Fig. 8, FAD exhibits a lower fluorescence intensity in the lesion than in the normal region. Therefore, recognizing the difference in fluorescence intensity of FAD enables distinction between normal region and lesion.

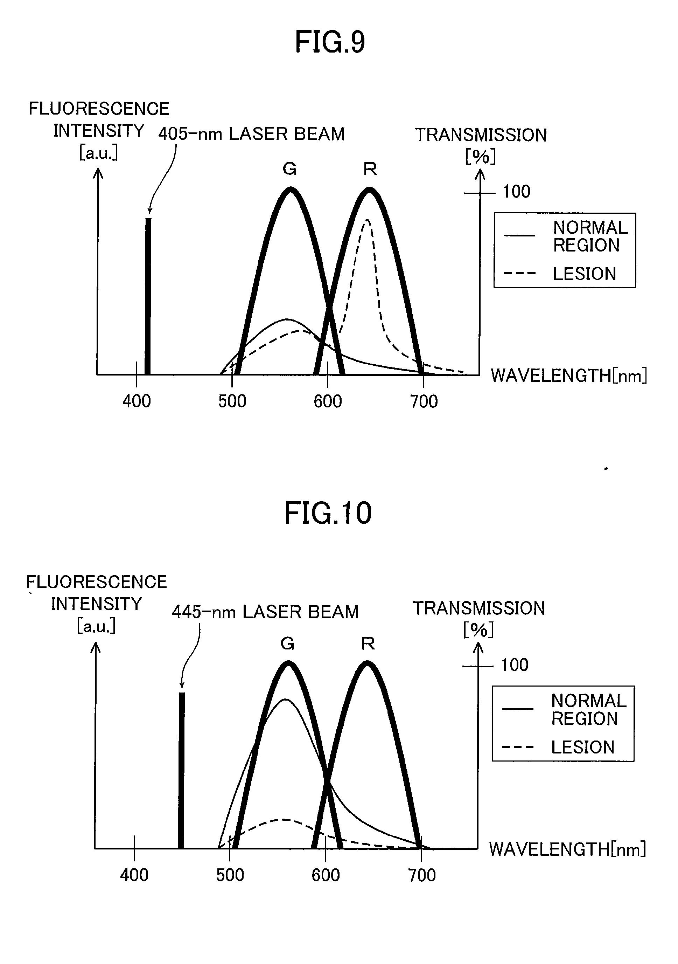

Figs. 9 and 10 are graphs where spectral transmission characteristics of color filters provided at the light receiving surface of the image sensor 58B are superimposed over the graphs of Figs. 7 and 8, respectively. The spectral transmission (%) of the color filters is shown on the vertical axis on the right side of the graph. The graphs also show central wavelengths 405 nm and 445 nm, respectively, of the laser beams used as excitation light for autofluorescence observation.

As these graphs illustrate, the green color filters are designed to transmit light having a wavelength range of 500 nm to 620 nm with the central wavelength at about 550 nm. Thus, autofluorescence emitted mostly from FAD is transmitted through the green color filters and undergoes photoelectric conversion through the image sensor 58B. The red color filters are designed to transmit light having a wavelength range of 580 nm to 700 nm with a central wavelength at about 630 nm. Thus, autofluorescence emitted mostly from porphyrin is transmitted through the red color filters and undergoes photoelectric conversion through the image sensor 58B.

Without limitation to the above, it is preferable that the green color filters transmit light having a wavelength range of 500 nm to 600 nm and the red color filters transmit light having a wavelength range of 600 nm to 700 nm.

As described above, the image sensor 58B is not provided with blue color filters. Thus, the design is so made that the laser beams for autofluorescence observation having central wavelengths of 405 nm and 445 nm are cut off and, are hence not allowed to undergo photoelectric conversion through the image sensor 58B.

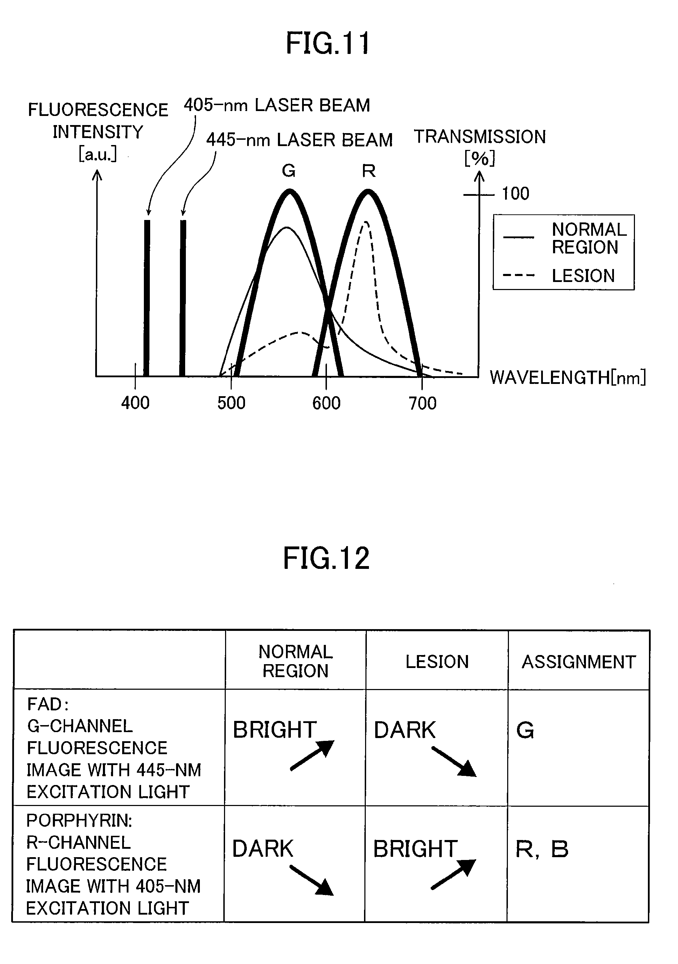

Fig. 11 is a graph where the spectral transmission characteristics of color filters are superimposed over a graph of fluorescence intensity distribution of autofluorescence when excitation light having central wavelengths of 405 nm and 445 nm are simultaneously emitted for illumination as excitation light for autofluorescence observation. The graph also shows central wavelengths 405 nm and 445 nm of the laser beams used as excitation light for autofluorescence observation.

It appears from this graph that when laser beams having central wavelengths of 405 nm and 445 nm are simultaneously emitted for illumination as excitation light for autofluorescence observation, there arise large differences in fluorescence intensity of autofluorescence in the lesion and the normal region between the autofluorescence having a wavelength near 550 nm emitted mostly from FAD and the autofluorescence having a wavelength near 630 nm emitted mostly from porphyrin.

The autofluorescence are dispersed through the green and the red color filters to obtain a green autofluorescence image and a red autofluorescence image. The green autofluorescence image permits distinction in fluorescence intensity of the autofluorescence emitted by FAD between the normal region and the lesion. Likewise, the red autofluorescence image enables distinction in fluorescence intensity of the autofluorescence emitted by porphyrin between the normal region and the lesion.

In the normal region, the fluorescence intensity of FAD increases while the fluorescence intensity of porphyrin decreases, whereas the relation reverses in the lesion, as illustrated in Fig. 12. As described above, the endoscopic diagnosis system 10 achieves pseudo color display by assigning the G channel image signal to the G channel, and the R channel image signal to the R and B channels. Thus, the autofluorescence image displayed in pseudo color by the endoscopic diagnosis system 10 represents the normal region in green and the lesion in magenta and, when compared with the individual autofluorescence images each obtained by imaging the autofluorescence emitted by FAD and porphyrin, represents the lesion in a further enhanced contrast, allowing easier recognition of the lesion.

Thus, the endoscopic diagnosis system 10 achieves acquisition of the autofluorescence images of autofluorescent substances excited by the individual excitation light having different wavelengths in sequential illumination but by simultaneous illumination by a plurality of excitation light having different wavelengths and dispersing the autofluorescence emitted from autofluorescent substances through green and red color filters to obtain green and red autofluorescence images. Therefore, no misalignment occurs between the green and red autofluorescence images, and the difference between normal region and lesion can be displayed with a high contrast, enabling an accurate distinction of a lesion. Another advantage is that no excitation light cut filer is required.

The autofluorescent substances used are not limited to porphyrin and FAD, provided that they are a combination of an autofluorescent substance having an autofluorescence intensity that is high in the lesion and low in the normal region and an autofluorescent substance having an autofluorescence intensity that is high in the normal region and low in the lesion. The number of autofluorescent substances for simultaneously emitting autofluorescence is not limited to two; for example, three or more excitation light having different wavelengths may be allowed to simultaneously illuminate a subject to cause three or more autofluorescent substances to emit light simultaneously.

The present invention is basically as described above.

While the invention has been described above in detail, the invention is by no means limited to the above embodiments, and various improvements and modifications may of course be made without departing from the spirit of the present invention.

## Claims

1. An endoscopic diagnosis system, comprising:
a light source for simultaneously emitting at least two excitation light having different central wavelengths for exciting at least two autofluorescent substances to generate autofluorescence, the at least two autofluorescent substances containing a first autofluorescent substance whose autofluorescence intensity is strong in a lesion and weak in a normal region and a second autofluorescent substance whose autofluorescence intensity is strong in a normal region and weak in a lesion;
color filters having spectral transmission characteristics that block the at least two excitation light and transmit the autofluorescence generated from the at least two autofluorescent substances;
image sensor for imaging autofluorescence generated, as the at least two excitation light simultaneously emitted from the light source illuminate a subject's region under observation, from the first and second autofluorescent substances contained in the region under observation and dispersed by the color filters to acquire first and second autofluorescence images; and
an image processor for image-processing the first and second autofluorescence images acquired by the image sensor and displaying the image-processed first and second autofluorescence images on a monitor.

2. The endoscopic diagnosis system according to Claim 1, wherein the image processor assigns the first autofluorescence image to B and R channels and the second autofluorescence image to a G channel to display images displayed on the monitor in pseudo color.

3. The endoscope diagnosis system according to Claim 2, further comprising:
a second light source for emitting white light;
second color filters having spectral transmission characteristics that transmit reflected light from the region under observation; and
a second image sensor for imaging the reflected light reflected from the region under observation and dispersed by the second color filters as white light emitted from the second light source illuminates the subject's region under observation to acquire a white light image;
wherein the image processor combines the image displayed on the monitor and the white light image acquired by the second image sensor and displays the combined image on the monitor.

4. The endoscopic diagnosis system according to any one of Claims 1 to 3, wherein the light source emits two excitation light having central wavelengths of 405 nm and 445 nm.

5. The endoscopic diagnosis system according to Claim 4, wherein the excitation light are laser beams.

6. The endoscopic diagnosis system according to any one of Claims 1 to 5, wherein the color filters comprise a green color filter that transmit light having a wavelength range of 500 nm to 600 nm and a red color filter that transmit light having a wavelength range of 600 nm to 700 nm.

7. The endoscopic diagnosis system according to any one of Claims 1 to 6, wherein the first autofluorescent substance is porphyrin and the second autofluorescent substance is FAD.
